Europäisches Patentamt

**European Patent Office**

Office européen des brevets

(11) Publication number: **0 092 914**
**A2**

## (12) EUROPEAN PATENT APPLICATION

(21) Application number: **83301787.4**

(22) Date of filing: **30.03.83**

(51) Int. Cl.³: **A 61 J 19/00**

(30) Priority: **26.04.82 US 372071**

(43) Date of publication of application:
**02.11.83 Bulletin 83/44**

(84) Designated Contracting States:
**BE CH DE FR GB IT LI NL**

(71) Applicant: **FALCON BRODY INC.**
**17975 Porto Marina Way**
**Pacific Palisades California 90272(US)**

(72) Inventor: **Johnson, Norman Eric**
**P.O. Box 1729**
**Big Bear Lake California 92315(US)**

(72) Inventor: **Falcon, Sanders Morris**
**1015, Third Street, No. 23**
**Santa Monica California 90403(US)**

(72) Inventor: **Norman, Michael**
**P.O. Box 3525**
**Big Bear Lake California 92315(US)**

(74) Representative: **Orchard, Oliver John**
**JOHN ORCHARD & CO. Staple Inn Buildings North High**
**Holborn**
**London WC1V 7PZ(GB)**

(54) Method and apparatus for collecting and washing sputum specimens for clinical diagnosis.

(57) A container assembly for collecting, transporting and washing sputum specimens for diagnostic assay in a hygienic and safe manner is provided with a chamber having a support for collected sputum, the chamber being provided with a removable, sealing lid, and a closeable drain; the removable tubular sleeve optionally provided between chamber and lid with a pair of laterally and vertically spaced tubular channels projecting from the side thereof, adapted to be connected to a vacuum source and to a catheter, respectively; the sputum being collected by either direct expectoration into the chamber or by the use of a suction catheter. Contaminants are removed from the sputum by agitation with a washing fluid on the support surface and subsequent separation through the support surface.

Fig.1.

./...

COMPLETE DOCUMENT

Croydon Printing Company Ltd.

Fig.5.

## BACKGROUND OF THE INVENTION

This invention relates to a method for washing sputum specimens, and to an apparatus for carrying that method into effective practice.

Pulmonary infection is a leading cause of mortality (fifth leading cause of death) and morbidity (ten percent of hospital admissions). The low diagnostic yield from routine laboratory "sputum" gram stain and culture is a recognized clinical problem and may be a factor in the continued high rate of morbidity and mortality from these diseases.

Routine laboratory gram stain and culture results from expectorated sputum are misleading because of oropharyngeal contamination of the sputum bolus as it exits through the supraglottic respiratory tract. While the infected sputum specimen contains only one responsible pathogen, the oropharyngeal cavity through which it passes is normally colonized by a complex and extensive microbial flora.

Many methods have been employed in an attempt to eliminate oropharyngeal contaminants from expectorated sputum gram stain and culture results. Although successful, none of these methods have gained routine acceptance due to invasiveness (lung aspiration, bronchoscopy, transtracheal aspiration), cost (liquifacation, dilution, homogenation), and time (manual sputum washing).

Washing expectorated sputum in normal saline or tap water results in improved sensitivity and specificity of gram stain and culture, rendering it comparable to specimens obtained via oropharyngeal-bypassing techniques such as transtracheal aspiration or bronchoscopy. Sputum washing therefore converts currently low-yield diagnostic gram stain and culture techniques into high-yield procedures without unnecessary invasiveness and cost.

Indeed, washed sputum gram stain immediately indicates the correct pathogen, and is subsequently corroborated by washed sputum culture, in 80 to 90 percent of cases.

Many conscientious physicians have been collecting and washing their own sputum specimens for years because their efforts have been rewarded with diagnostic information far superior to routine laboratory results from unwashed specimens. However, sputum washing methods have - until now - been considered too tedious and time-consuming to gain acceptance in mainstream physician and laboratory practice.

The prior art contains many references to the desirability of performing certain washing procedures and tests on sputum specimens, and provides certain specific containers in which sputum may be collected and in which certain procedures may be performed with minimal exposure of laboratory personnel to the contents.

An example of the former is a paper by John G. Bartlett and Sydney M. Finegold in the "American Review of Pulmonary Disease," Volume 117 (1978), p. 1019ff., entitled "Bacteriology of Expectorated Sputum with Quantitative Culture and Wash Technique compared to Transtracheal Aspirates." Bartlett and Finegold describe a manual technique for washing sputum specimens utilizing "sterile tea strainer(s)" to selectively retain the sputum in the expectorant and conclude by saying "The methods used in this study would appear to be unrealistic for routine use by clinical laboratories. It is conceivable that a more simplified technique could be devised using the same principals(sic)."

An example of the latter class of prior art publications is U.S. patent 3,518,164 to J.P. Andelin, et al., describing a "Diagnostic Sputum Collection System" in the form of a container suited for hygienic reception and transportation of expectorated sputum specimens, but not adaptable to suction or washing techniques.

It is, therefore, an object of the invention to provide a process for the separation of normally sterile sputum from contaminants introduced in its' collection.

It is also an object of this invention to provide a device for collection of sputum specimens in which the sputum may be separated from its contaminants.

It is a further object of the invention to provide strainer means integrated with said specimen collection container, so as to provide for the pre-assay washing of the collected specimen within the container provided, and means for draining the wash solution therefrom.

It is also an object of the invention to provide means for readily identifying the specimen in said container and for coding the desired analytical and laboratory procedures by means of a label affixed to a planar surface thereof.

It is a fundamental object of the invention to teach the performance of a method - employing the specimen collection container defined hereinabove - for collecting sputum for laboratory examination, for hermetically enclosing it for transfer and handling, for washing the specimen contained therein in an aqueous or saline solution, and for sampling the specimen for laboratory studies such as stains and cultures.

## SUMMARY OF THE INVENTION

The foregoing objects - and other objects and advantages of the invention which shall become apparent from the detailed description of the method and of the preferred embodiment of the apparatus thereof, below - are attained with the aid of a sputum sample receptacle/container comprised of a sterile chamber with a removable, close-fitting lid and a basal surface developed as a fine-meshed screen or perforated plate.

The receptacle of the sample receiving device may also be provided above the support plate with a removable sleeve provided with ports penetrating the sidewall thereof at two different locations spaced in a vertical direction, as well as laterally, from one another. One of these ports is designed to be connected to a catheter by means of a flexible tube, while the other is adapted to be connected to a source of vacuum, such as is conventionally provided in medical offices and hospital examination rooms. The lower port is dedicated to the catheter line, while the upper one is used for developing the vacuum by means or which sputum may be drawn up the catheter into the receiving chamber of the container.

Where the sample is to be obtained by expectoration, the lid of the receiving chamber may be removed for the direct deposition therein of the sample, without use of the removable suction port sleeve.

At mid chamber is placed a support permeable to wash fluid but relatively impermeable to sputum, a jelled fluid suspension of high viscosity. The basal chamber portion contains a sealable discharge orifice. The function of the lower portion of the chamber is to receive the cleansing fluid during the wash cycle, to be discharged through the aforementioned orifice.

Auxiliary components of the apparatus include a flexible elastomeric tube adapted to be slipped over the suction and catheter ports, so as to maintain a sterile, sealed internal volume before, and after, the sample acquisition period, and a label, preferably affixed to the upper, flat surface of the removable lid, preprinted with the information necessary to easily code thereunto the identity of the patient and the information necessary for the instruction of laboratory personnel in performing the desired tests.

Sample acquisition is straightforward when the oral expectorant method is used: the suction-port sleeve and lid are removed and the sputum deposited directly on the support surface of the receiving chamber, with the lid - with a tight-fitting snap-closure in the preferred embodiment of the apparatus - replaced after a sufficient quantity of sputum has been collected.

0092914

For suction-assisted sample acquisition, the flexible tube interconnecting the two side ports of the receiving chamber sleeve is removed and the catheter conduit attached to the lower one, a source of vacuum being connected to the upper. The catheter is next inserted into the trachea of the patient and sputum is drawn up into the receiving chamber by vacuum suction. The relative elevation of the suction port prevents the accidental entrainment of the collected specimen towards the vacuum source. When sufficient material has been deposited in the recepticle, the suction port sleeve is entirely removed and the lid is replaced on the chamber.

At this time the clinician collecting the sample makes the appropriate entries on the label atop the chamber lid, and forwards the now hermetically sealed container to the assay laboratory.

At the laboratory the technician will wash the sample by removing the lid and filling the container to a pre-determined and marked "wash-line" with tap water or saline solution. The wash-line is approximately at the level of the lid.

By swirling the reclosed container, the saliva adhering to the sputum on its passage through the oropharynx, as well as any other contaminants, are separated from the sputum by dissolution in the wash. The washing procedure is repeated as necessary, with the wash solution containing oropharyngeal contaminants drained through the drain opening at the conclusion of each wash cycle; the drain being reclosed prior to the beginning of the next wash cycle.

After the sample has been washed, the procedures defined by the instructions on the label affixed to the cover are to be performed, and the results conveyed to the physician.

To ensure the reliability and traceability of each sample, and to prevent accidental contamination through the loss of such components, loose portions of the container – the drain cap and lid are attached to the main container by flexible straps.

BRIEF DESCRIPTION OF THE ACCOMPANYING DRAWING

The preferred embodiment of the apparatus of the invention, and the sequential steps of the method associated therewith, are described in detail below with reference to the accompanying drawing, wherein:

Figure 1 is a perspective view of the sputum sample collecting container of the invention in a state corresponding to the pre-use mode, with the drain cap in place and the suction port sleeve in place with the flexible tube sealing the side ports slipped over the stub channels leading to these ports;

Figure 2 is a partial section of the embodiment of Figure 1, showing the interposition of the perforated sputum support surface plate in the chamber thereof;

Figure 3 is a side view of an alternate embodiment of the container with threaded joints between the upper suctionport sleeve and lower chamber, as well as between the upper sleeve and the lid, enclosed in a sterile shipping bag to prevent its contamination prior to actual use;

Figure 4 is a partial, lateral section – taken along section line 4-4 in Figure 3 – through the embodiment of Figure 3, particularly showing the recessed stub-channels of the sleeve ports and the use of a screened panel or perforated plate as the sample support platform; and

Figure 5 is a sequential diagram showing the sample collection container prior to use, the alternate methods of securing sputum samples and their deposition into the container, the closure of the container for transport to the laboratory, the admission of wash solution into the container, the washing of the sample, and the draining of the wash solution.

DESCRIPTION OF THE PREFERRED EMOBIDMENT AND METHOD

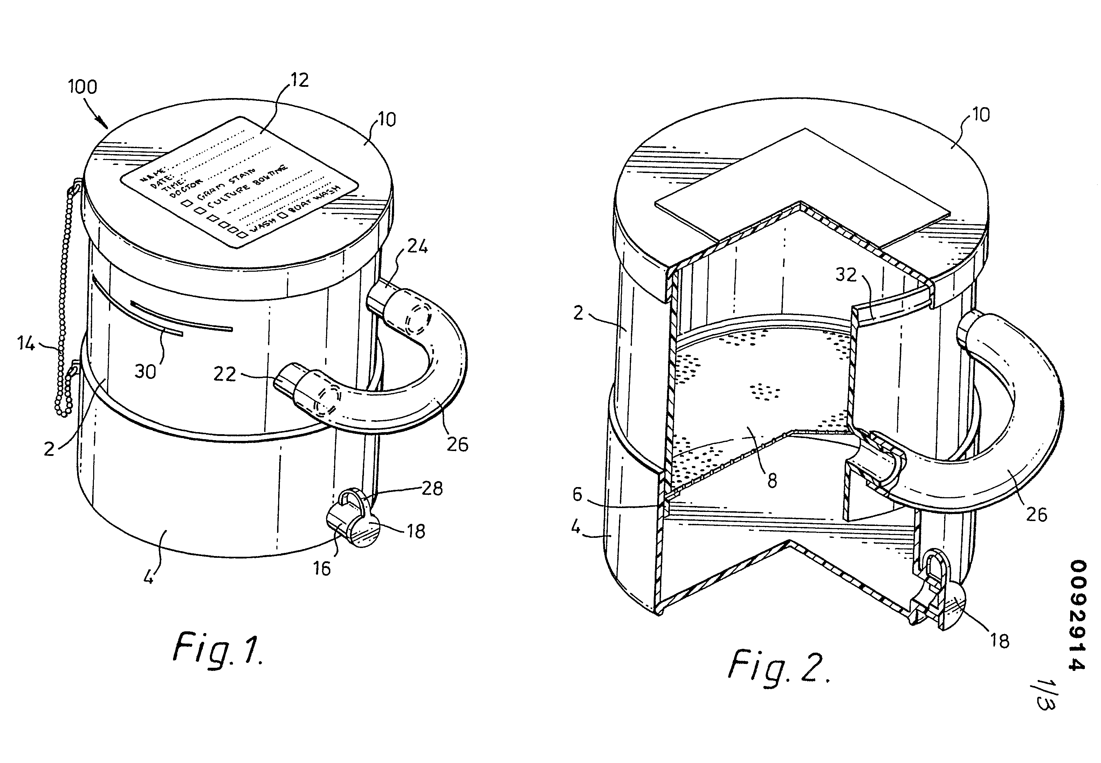

The perspective view of Figure 1 shows a sputum sample container 100 of the invention with a tubular sleeve 2, and a drain compartment 4. The drain compartment 4 is provided with an internal, circumferential shelf 6, upon which a screen or perforated plate –suitably of stainless steel wire mesh or a woven plastic material – is placed to form a sputum sample support surface 8. The mesh openings or perforations of the sample support 8 are so selected that a liquid with the fluidity of water will readily pass therethrough, but the viscous gobs of sputum will be safely supported thereon.

The shelf 6 and the drain compartment are permanently joined, by means or a suitable adhesive or by sonic welding applicable to the material from which they are fabricated, suitably a softer grade of polyethylene or polypropylene. A Lid 10 is also provided, in sealing engaging over the upper edge of the sleeve 2 or chamber 4 - suitably by means of a snap closure molded into the mating surfaces of the two components.

A label 12, preprinted with essential information spaces for the patient's identification and for the laboratory procedures to be performed, is securely mounted on the upper surface of the lid 10. Suitably the label is treated and printed in such a manner that it will readily accept markings by all forms of writing implement - pencils, pens, felt-tip markers, chinagraph markers, etc. - and retain such information even if accidentally wetted. A hinge, 14 interconnects the lid 10 with the body of the container, to prevent accidental loss of the former with its information.

Two ports - spaced laterally and vertically - penetrate the sidewall of the suction sleeve 2 with tubular stub channels 22 and 24 projecting therefrom. A flexible, elastomeric tube 26 is pressed over the channels 22 and 24 with either end, to form a sealing closure.

A drain port in chamber 4 is similarly provided with a protruding stub channel 16 and may be closed by a tight-fitting plug 18 with an integral, hinge 28.

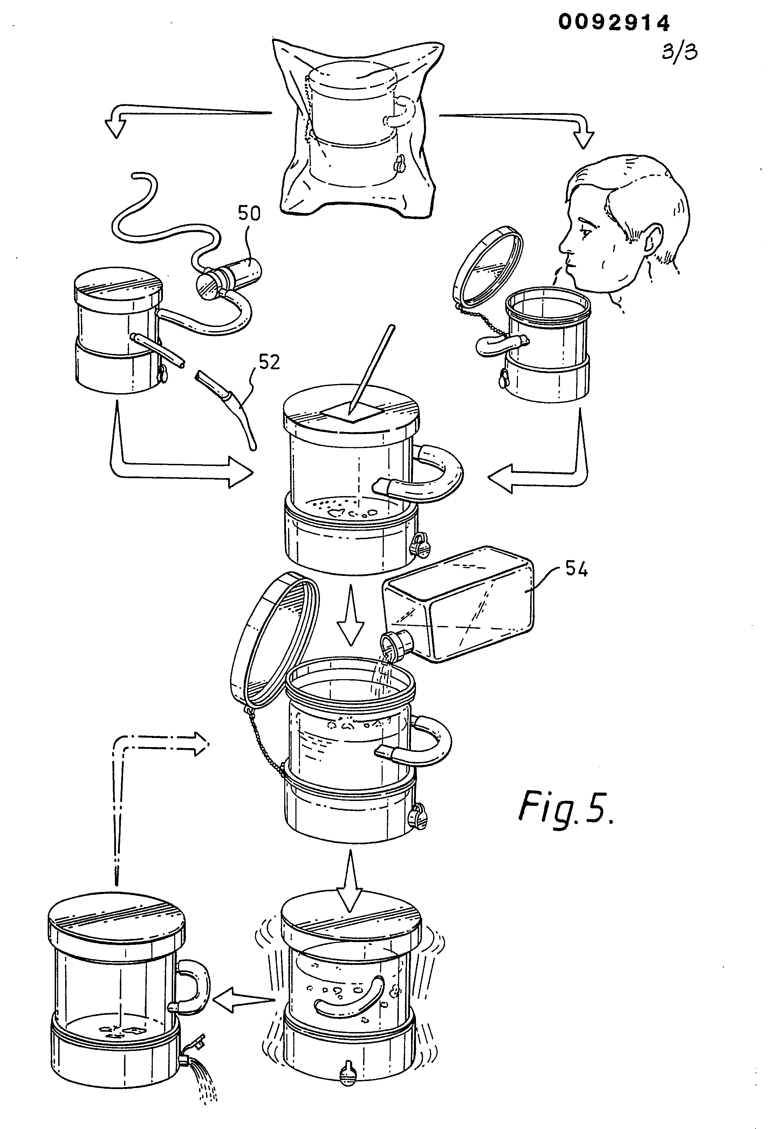

Molded-in marking lines 30 establish a predetermined level for wash water - the procedure to be more fully explained with reference to the sequential diagram of Figure 5 - in the container.

The partially sectioned perspective view of Figure 2 represents the container assembly 100 of Figure 1, and exposes the internal construction thereof. In particular, the manner in which the sample support screen or plate 8 is placed over the shelf 6 and entrapped by the interaction of container portion is clearly seen, as well as the sealing interaction of the drain plug 18 and the tube 26 with respect to the appropriate openings into the container. An interacting ridge-and-groove snap closure between the chambers 2 and 4 and the lid 10 is indicated at 32.

In use with a catheter and vacuum source for the obtaining of the sputum sample (refer to figure 5) the lower opening 22 into the suction sleeve 2 is used for the catheter tube, while the upper port 24 is connected to the vacuum pump. The vertical and lateral separation of the two ports 22 and 24 ensures that the sample will be deposited onto the support screen 8, and not drawn into port 24 toward the vacuum source therethrough.

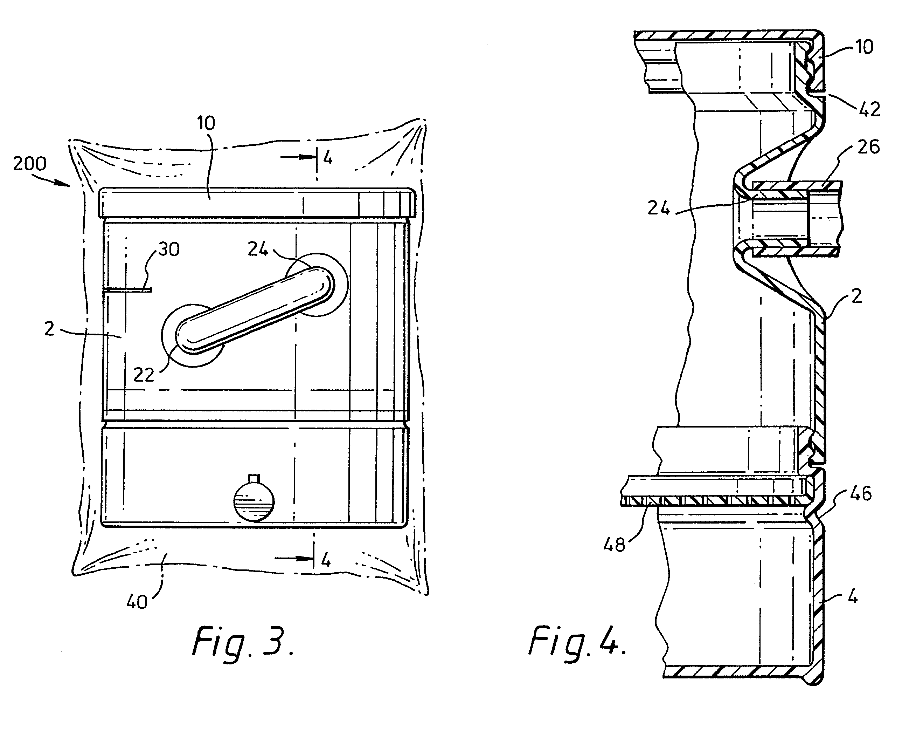

The side view of Figure 3 represents another embodiment 200 of the sputum sample container of the invention, differing from the unit shown in Figure 1 in minor details of construction. The components indexed with the same numerals as in Figure 1 and 2 serve identical functions in both embodiments 100 and 200.

In Figure 3, the assembly 200 is seen to be enclosed in a hermetically sealed bag 40 - made from impervious paper or plastic sheathing, and serving to prevent contamination of the sample container after sterilization and before use.

The fragmentary sectional view - taken along section line 4-4 in Figure 3 - of Figure 4 also shows the sample container 200 including a threaded interconnection 42 between the suction port sleeve 2 and the lid 10 and a similar connection between the drain compartment 4 and the lid. A sample support sheet 48 is provided - in place of the screen 8 of Figure 1 and 2 - made from a suitable rigid or semi-rigid material and pierced by a plurality of small orifices which perform the same function of passing liquid solutions but preventing the entry of sputum as the mesh openings of the screen 8. The sample support 48 is locked in position between an inwardly projecting ridge 46 in the wall of the drain compartment 4 and the bottom edge of the suction sleeve 2.

The stub channels 22 and 24 associated with the side ports in the wall of the suction sleeve 2 are protected by concentric indentations in the wall of the latter, thereby reducing the hazard that the cross-over tube 26 - or the connections to the catheter and vacuum source in operational use - will be accidentally dislodged therefrom.

The method of the invention - illustrated in the sequential diagram of Figure 5, comprising seven separate perspective views - foresees two alternative procedures for obtaining sputum samples, by expectoration and by suction.

The initial step in the method is the unwrapping and uncovering of the sample receiving chamber, by the removal of the lid 10 and sleeve 2, when the sample is to be acquired by having a patient expectorate directly onto the support surface formed by a screened or perforated support plate at the base of the receptacle.

The initial step in the suction method requires the unwrapping of the sample container assembly from its protective bag and the removal of the cross-over seal tube 28 from the projecting channel stubs associated with the side ports in the wall of sleeve 2. After the removal of the tube 28, the upper port is connected, by means or a suitable flexible tube, to a vacuum pump 50 - or to a centralized vacuum/suction line communicating with a vessel maintained at below-atmospheric pressure. The lower port is similarly connected to a catheter 52, or its equivalent, which may be inserted into the patient's trachea to secure appropriate samples of sputum for examination.

At the conclusion of the sample-acquisition step by either method contemplated, the sample container assembly is closed - by removal of the sleeve 2 and replacement of the lid 10, as required - and the label thereon is marked with the necessary information for laboratory handling. The sealed sample container is then transferred to the assay laboratory.

The next step in the performance of the method will normally take place in the laboratory and involves the washing of the sputum specimen obtained. A wash solution - either tapwater, or normal saline - is poured into the container, from a bottle 54 or other source, after the temporary removal of the lid 10 therefrom, until the liquid level in the container is at the wash-line mark 30. The container is then reclosed into a watertight condition, by the reinstallation of the lid 10.

The entire assembly is then put into an oscillatory motion — either by manual shaking or by placement into a shaking unit adapted for such prupose. The rapid movement of the wash solution within the sample container is only marginally impeded by the sample support with its high porosity, so that the entire volume of solution within the container is available for the removal and storage of water soluble or suspendable contaminants on the surface of the sample.

After a reasonable period of agitation — suitably of the order of one minute — the motion is stopped and the wash solution is allowed to drain into a disposal sink through the removal of plug 18 from the drain orifice at the bottom of the drain compartment 6.

The drain plug 18 is re-inserted next, the lid 10 removed and the wash cycle — encompassing the steps of adding a wash solution, reclosing the container, and agitating the wash solution around the sample reposing on the support surface — is repeated as necessary.

Upon the completion of the washing of the sample, sputum may be removed from the screen.

The wash lines 30 have been shown on the sleeve 2, because of the location of the support 8 in chamber 4. In practice, if the sleeve 2 remains on the base during washing, the ports 22 and 24 should be sealed with the tube 26. Alternatively, the support 8 may be lowered in the chamber 4, and the wash level lines provided on the exterior of the chamber 4 rather than the sleeve 2.

The invention has been described with reference to its simplest extent, additional procedures may be undertaken with the sample still in the container of the invention after the completion of the wash cycle, for example, or steps may be interposed into the described process elsewhere. Such additional steps shall be deemed ancillary to the described sequence and encompassed by the disclosure herein.

The apparatus of the invention has been described with reference to its preferred embodiment along with variant constructional details therein. Where features are separately described with reference to different embodiments, it is understood that they, or other mechanical variants for attaining a similar function with cognate means, may be utilized in varying combinations and are deemed to be encompassed by the disclosure herein. The scope of the invention is solely delimited by the appended claims.

We claim:

1.  Container assembly for sputum samples collected for laboratory analysis, comprising:

an upright, circumferentially enclosed receptacle for said samples having a drain chamber provided with a water-tight bottom closure;

a removable lid, sealingly encompassing an upper edge of said receptacle;

a sputum support surface above said bottom closure, and

a closeable drain opening proximate to the base of said receptacle piercing a sidewall thereof below said support.

2.  The device of claim 1, further comprising a sleeve interposed between said lid and said receptacle, said sleeve containing a pair of orifices piercing the circumferential enclosure thereof; with said orifices spaced circumferentially and vertically from one another.

3.  The container assembly of claim 2, wherein said removable lid obtains its sealing engagement with said upper edge of said sleeve by means of interacting snap joint means incorporated in said components.

4.  The container assembly of claim 2, wherein said removable lid is secured to the upper edge of said receptacle by means of a threaded connection.

5.  The assembly of claim 1, further comprising a label adapted for receiving written information on a surface thereof.

6.  The container assembly of claim 1, wherein said sputum support surface is defined by a perforated plate.

7. The container assembly of claim 1, wherein said sputum support surface is defined by a rigid planar member with a plurality of orfices.

8. The method which comprises collecting a sputum sample on a support pervious to a washing fluid and relatively impervious to the sputum, agitating said sputum in the presense of said wash fluid and separating sputum from wash through said support.

9. The method of claim 8 wherein said sample is collected by vacuum means from a patient.

Fig. 1.

Fig. 2.

0092914

1/3

Fig. 3.

Fig. 4.

50

52

54

Fig.5.